# FASCICULE DE BREVET EUROPEEN

(11) **EP 1 779 895 B1**
(45) Date de publication et mention de la délivrance du brevet: **06.03.2013**
(21) Numéro de dépôt: 06291668.9
(22) Date de dépôt: 26.10.2006
(51) Int. Cl.: A61Q 5/06, A61K 8/81, A61K 8/91

(54) **Composition cosmétique comprenant au moins un polymère fixant particulier et au moins un polymère sulfoné**
Kosmetische Zusammensetzungen enthaltend mindestens ein fixierendes Polymer und ein Sulfonpolymer
Cosmetic composition comprising at least one fixing polymer and at least a sulfonated polymer

(30) Priorité: 28.10.2005 FR 0511090
(43) Date de publication de la demande: 02.05.2007
(73) Titulaire: L'Oréal, 75008 Paris (FR)
(72) Inventeur: Annotel, Christine, 75019 Paris (FR); Laurent, Ludivine, 92400 Courbevoie (FR)
(74) Mandataire: Zapalowicz, Francis

(56) Documents cités:
- EP-A- 0 611 566
- EP-A- 1 632 270
- WO-A-2006/030113
- FR-A- 2 819 177
- US-A- 5 686 067
- US-B1- 6 451 299

## Description

La présente invention concerne une composition cosmétique pour le traitement des fibres kératiniques, en particulier des fibres kératiniques humaines telles que les cheveux, comprenant au moins un polymère fixant particulier et au moins un polymère sulfoné particulier, une utilisation de cette composition pour la mise en forme et/ou le maintien de la coiffure ainsi qu'un procédé de traitement cosmétique la mettant en oeuvre.

Dans le domaine du coiffage, en particulier parmi les produits capillaires destinés à la mise en forme et/ou au maintien de la coiffure, les compositions capillaires sont généralement constituées d'une solution le plus souvent alcoolique ou aqueuse et d'un ou de plusieurs polymères fixants en mélange avec divers adjuvants cosmétiques.

Ces compositions peuvent se présenter sous forme de gels ou de mousses capillaires qui sont généralement appliquées sur des cheveux mouillées avant d'effectuer un brushing ou un séchage.

En particulier, les gels capillaires sont notamment constitués d'un ou de plusieurs polymères épaississants ou agents gélifiants en association avec un ou plusieurs polymères fixants qui ont le plus souvent pour fonction de former un film à la surface des fibres kératiniques à fixer afin de réaliser des soudures entre celles-ci.

Ces gels capillaires doivent de préférence présenter une texture glissante qui leur permet de s'étaler facilement sur la chevelure sans laisser une sensation collante ou de frein lors de l'application.

Par ailleurs, ces gels capillaires doivent de préférence présenter également une texture fondante qui leur permet d'être plus facilement absorbé à la surface de la chevelure.

Cependant, l'association de polymères fixants avec un ou plusieurs polymères épaississants ou agents gélifiants dans des gels capillaires donne lieu, plus ou moins rapidement, à la formation de résidus inesthétiques sur la chevelure issus de la dégradation partielle des soudures.

Il existe donc un réel besoin de trouver des compositions cosmétiques, notamment pour le coiffage, qui permettent de minimiser la formation de résidus inesthétiques sur les cheveux, tout en leur conférant un niveau élevé de fixation pour obtenir une mise en forme et/ou un maintien de la coiffure satisfaisant.

De manière surprenante et avantageuse, la Demanderesse vient de découvrir qu'en associant un polymère fixant particulier et un polymère sulfoné particulier comportant au moins un motif dérivé d'un monomère à insaturation éthylénique à groupement sulfonique, il était possible de minimiser la formation des agrégats sur la chevelure et d'obtenir des propriétés cosmétiques satisfaisantes.

Par ailleurs, une telle association permet également d'obtenir un gel de coiffage qui présente une texture satisfaisante, c'est-à-dire une texture fondante et glissante.

La présente invention a donc notamment pour objet une composition cosmétique pour le traitement des fibres, en particulier des fibres kératiniques humaines telles que les cheveux, comprenant une telle association.

Un autre objet de la présente invention consiste en une utilisation de la composition selon l'invention pour la mise en forme et/ou le maintien de la coiffure.

L'invention concerne également un dispositif aérosol comprenant une composition cosmétique selon l'invention.

Un autre objet encore est un procédé de traitement cosmétique mettant en oeuvre la composition selon l'invention

D'autres objets, caractéristiques, aspects et avantages de l'invention apparaîtront encore plus clairement à la lecture de la description et des exemples qui suivent.

Selon l'invention, la composition cosmétique pour le traitement des fibres kératiniques, en particulier des fibres kératiniques humaines telles que les cheveux, comprend, dans un milieu cosmétiquement acceptable :
- au moins un polymère fixant anionique comprenant au moins en tant que monomères un vinyl lactame, un acide carboxylique à insaturation éthylénique et un acrylate ou un méthacrylate d'alkyle, la portion alkyle comportant au moins 6 atomes de carbone, et
- au moins un polymère sulfoné partiellement ou totalement neutralisé, comprenant au moins un motif dérivé d'un monomère à insaturation éthylénique à groupement sulfonique, selon la revendication 1.

Par polymère fixant, on entend tout polymère permettant de conférer une forme ou de maintenir une forme ou une coiffure donnée.

L'acide carboxylique à insaturation éthylénique est choisi de préférence parmi l'acide acrylique, l'acide méthacrylique, l'acide itaconique ou l'acide crotonique.

De préférence l'acide carboxylique à insaturation éthylénique est l'acide acrylique ou méthacrylique. Encore plus préférentiellement, cet acide carboxylique à insaturation éthylénique est l'acide acrylique.

Le vinyl lactame est en particulier la vinyl pyrrolidone.

L'acrylate ou le méthacrylate d'alkyle comportant au moins 6 atomes de carbone, est de préférence un ester acrylique ou méthacrylique comprenant un radical alkyle comportant de 8 à 18 atomes de carbone.

Les radicaux alkyle sont choisis de préférence parmi les radicaux 2-éthyl hexyle, octyle, lauryle et stéaryle.

Ces polymères peuvent être éventuellement réticulés.

Les polymères fixants anioniques utilisables dans la composition selon l'invention peuvent être préparés par exemple selon le procédé décrit dans le brevet US 5 015 708.

Le document US-B1-6 451 299 décrit des compositions fixant la coiffure à base de terpolymère de polyvinylpyrrolidone, d'acide acrylique et de laurylméthacrylate.

Les polymères fixants anioniques utilisables dans la composition selon l'invention comprennent, de préférence, en tant que monomères de la vinylpyrrolidone, de l'acide acrylique et du méthacrylate de lauryle.

Un polymère particulièrement préféré est le polymère commercialisé sous la dénomination « Acrylidone LM» par la société ISP qui est un terpolymère de vinylpyrrolidone (68% en poids) /d'acide acrylique (23% en poids) et de méthacrylate de lauryle (9% en poids).

Le(s) polymère(s) fixant(s) anionique(s) à base de vinyl lactame, d'acide carboxylique à insaturation éthylénique et d'acrylate ou de méthacrylate d'alkyle est ou sont notamment présent(s) dans la composition à une concentration allant de 0,05 à 30% en poids, de préférence à une concentration allant de 0,1 à 20% en poids, et encore plus préférentiellement à une concentration allant de 0,5 à 10% en poids, par rapport au poids total de la composition.

Les polymères sulfonés selon l'invention sont choisis parmi les poly(acide 2-acrylamido 2-méthylpropane sulfonique) réticulés et neutralisés à au moins 90 % comprenant :
(a) de 90 à 99,9 % en poids de motifs de formule générale (I) suivante : 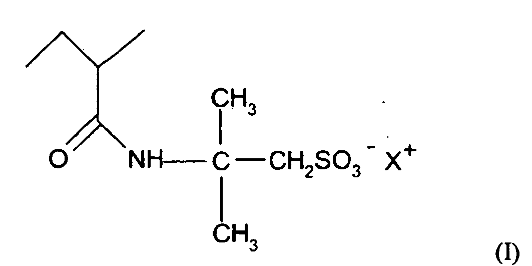 dans laquelle X⁺ est l'ion ammonium ;
(b) de 0,01 à 10 % en poids de motifs réticulants provenant d'au moins un monomère ayant au moins deux doubles-liaisons oléfiniques ; les proportions en poids étant définis par rapport au poids total du polymère.

De préférence, les polymères poly(acide 2-acrylamido 2-méthylpropane sulfonique) réticulés et neutralisés à au moins 90 % comprennent de 98 à 99,5 % en poids de motifs de formule (I) et de 0,2 à 2 % en poids de motifs réticulants.

Plus particulièrement, 90 à 100 % mole des cations sont des cations NH₄⁺ et 0 à 10 % mole sont des protons.

Les monomères de réticulation ayant au moins deux doubles liaisons oléfiniques peuvent être choisis par exemple parmi le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl-éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA).

Les polymères poly(acide 2-acrylamido 2-méthylpropane sulfonique) réticulés et neutralisés à au moins 90 % présentent une viscosité mesurée au viscosimètre Brookfield, mobile 4, à une vitesse de rotation de 100 tours/minutes, dans une solution d'eau à 2 % et à 25°C, supérieure ou égale à 1 Pa.s (10 poise; 1000cps).

De préférence, les polymères poly(acide 2-acrylamido 2-méthyl propane sulfonique) réticulés présentent une viscosité mesurée au viscosimètre Brookfield, mobile 4, à une vitesse de rotation de 100 tours/minutes, dans une solution d'eau à 2 % et à 25°C, allant de 5 Pa.s à 40 Pa.s (50 à 400 poise; 5000 à 40000 cps) et plus particulièrement de 6,5 Pa.s à 35 Pa.s (65 à 350 poise; 6500 à 35000 cps).

Les polymères sulfonés utilisables dans la composition cosmétique conforme à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH (2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.

Par ailleurs, en utilisant la polymérisation par précipitation dans le tert-butanol, il est possible d'obtenir une distribution de la taille des particules du polymère sulfoné particulièrement favorable pour ses utilisations.

La distribution de la taille des particules du polymère peut être déterminée par exemple par diffraction laser ou analyse d'image.

Une distribution intéressante pour ce type de polymère sulfoné et déterminée par analyse d'image est la suivante : 60,2% inférieur à 423 microns, 52,0% inférieur à 212 microns, 26,6% inférieur à 106 microns, 2,6% inférieur à 45 microns et 26,6% supérieur à 850 microns.

La réaction peut être conduite à une température comprise entre 10 et 150°C, soit à pression atmosphérique, soit sous pression réduite. Elle peut aussi être réalisée sous atmosphère inerte, et de préférence sous azote.

Par exemple, les poly(acide 2-acrylamido 2-méthyl propane sulfonique) réticulés peuvent être obtenus selon un procédé de préparation comprenant les étapes suivantes :
(a) on disperse ou on dissout le monomère acide 2-acrylamido 2-méthyl propane sulfonique sous forme libre dans une solution de tertio-butanol ou d'eau et de tertio-butanol ;
(b) on neutralise la solution ou la dispersion de monomère acide 2-acrylamido 2-méthyl propane sulfonique obtenue au cours de l'étape (a) par l'ammoniaque NH₃, dans une quantité permettant d'obtenir un taux de neutralisation des fonctions acides sulfoniques du polymère allant de 90 à 100 % ;
(c) on ajoute à la solution ou dispersion en (b) le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Les polymères sulfonés conformes à l'invention sont de préférence présents en une quantité allant de 0,05 % à 20 % en poids, plus préférentiellement entre 0,1 % et 10 % en poids et encore plus préférentiellement de 0,5 % à 5 % en poids par rapport au poids total de la composition de traitement capillaire.

De préférence, le rapport pondéral entre le ou les polymères fixants anioniques de l'invention et le ou les polymères sulfonés de l'invention est supérieur à 1.

La composition cosmétique selon l'invention peut comprendre en outre un ou plusieurs adjuvants cosmétiques choisis parmi les polymères fixants autre que ceux de l'invention, les silicones sous forme soluble, dispersées, micro ou nano-dispersées, les agents épaississants non polymériques, des polymères épaississants ou agents gélifiants non sulfonés, les agents tensioactifs cationiques, anioniques, amphotères, non ioniques, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents anti-corrosion, les corps gras tels que les huiles végétales, animales, minérales et synthétiques, les agents réducteurs ou antioxydants, les agents oxydants.

L'homme du métier veillera à choisir les éventuels adjuvants et leur quantité de manière à ce qu'ils ne nuisent pas aux propriétés des compositions de la présente invention.

De préférence, le ou les adjuvants cosmétiques sont présents à une concentration allant de 0,001 à 50 % en poids par rapport au poids total de la composition.

Par milieu cosmétiquement acceptable, on entend un milieu compatible avec les matières kératiniques et en particulier les cheveux.

Le milieu cosmétiquement acceptable peut être un milieu alcoolique, aqueux ou hydroalcoolique. Ainsi le milieu peut notamment être constitué uniquement par de l'eau ou de l'alcool ou par un mélange d'eau et d'un ou de plusieurs solvants cosmétiquement acceptables tels que les alcools inférieurs en C₁-C₄, les polyols, les monoéthers de polyols et leurs mélanges. De préférence, l'alcool est l'éthanol.

Les compositions conformes à l'invention peuvent être conditionnées dans un pot, dans un tube, un flacon pompe ou dans un dispositif aérosol usuel en cosmétique.

Les agents propulseurs utilisés dans les systèmes aérosols selon l'invention peuvent être choisis parmi l'air, l'azote, le gaz carbonique, le diméthyléther, les alcanes en C₃ à C₅, le 1,1-difluoroéthane et leurs mélanges.

La présente invention concerne également un dispositif aérosol comprenant la composition décrite ci-dessus et un moyen de distribution de cette composition.

La présente invention concerne aussi un procédé de traitement cosmétique des cheveux, par exemple de coiffage, qui consiste à appliquer une quantité efficace d'une composition décrite ci-dessus, sur les cheveux secs ou humides, à rincer ou non après un éventuel temps de pose ou après un éventuel séchage.

De préférence, la composition selon l'invention se présente sous forme d'un gel présentant une viscosité d'au moins 200 cps mesurée à 25 °C avec un rhéomètre RS600 THERMOELECTRON à un taux de cisaillement de 1s⁻¹.

De manière générale, la composition cosmétique présente une viscosité comprise entre 0,2 Pa.s et 100 Pa.s (2 à 1000 poise) à 25 °C, (200 et 100000 cps), de préférence entre 0,5 Pa.s et 50 Pa.s (5 et 500 poise) à 25°C (500 et 50000 cps), et encore plus préférentiellement entre 0,8 Pa.s et 30 Pa.s (8 et 300 poise) à 25°C (800 et 30000 cps) à un taux de cisaillement de 1s⁻¹ pouvant être mesurée à l'aide du rhéomètre RS600 de THERMOELECTRON.

La présente invention concerne également l'utilisation d'une composition cosmétique pour la mise en forme et/ou le maintien de la coiffure.

L'exemple suivant est donné à titre illustratif de la présente invention.

### EXEMPLE

On prépare le gel de coiffage selon l'invention à partir des composés suivants :
- Terpolymère vinylpyrrolidone/acide acrylique/méthacrylate de lauryle, proposé par la société ISP sous la dénomination Acrylidone LM 1 %
- Poly(acide 2-acrylamido 2-méthylpropane sulfonique) réticulé et neutralisé par de l'ammoniaque, proposé la société CLARIANT sous la dénomination Hostacerin AMPS 0,6 %
- Conservateurs qs
- Eau qsp 100 %

Les pourcentages de chacun des composés dans les gels de coiffage selon l'invention sont calculés en poids par rapport au poids total de la composition.

On obtient un gel de coiffage ayant une viscosité de 15 poise (1500 cps) à 25°C à un taux de cisaillement de 1s-¹ qui présente, après séchage, une texture glissante et fondante qui est appliquée facilement sur la chevelure.

On constate que la chevelure présente, après séchage, peu voire pas de résidus à la surface de la chevelure.

## Revendications

1. Composition cosmétique pour le traitement des fibres kératiniques, en particulier des fibres kératiniques humaines telles que les cheveux, **caractérisée par le fait qu'**elle comprend, dans un milieu cosmétiquement acceptable,
- au moins un polymère fixant anionique comprenant au moins en tant que monomères un vinyl lactame, un acide carboxylique à insaturation éthylénique et un acrylate ou un méthacrylate d'alkyle, la portion alkyle comportant au moins 6 atomes de carbone, et
- au moins un polymère sulfoné partiellement ou totalement neutralisé, comprenant au moins un motif dérivé d'un monomère à insaturation éthylénique à groupement sulfonique, ledit polymère sulfoné étant choisi parmi un poly(acide 2-acrylamido 2-méthyl propane sulfonique) réticulé et neutralisé à au moins 90 % comprenant :
(a) de 90 à 99,9 % en poids de motifs de formule générale (I) suivante : 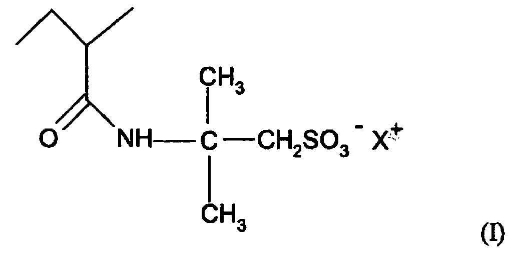 dans laquelle X⁺ est l'ion ammonium ;
(b) de 0,01 à 10 % en poids de motifs réticulants provenant d'au moins un monomère ayant au moins deux doubles-liaisons oléfiniques ; les proportions en poids étant définis par rapport au poids total du polymère.

2. Composition cosmétique selon la revendication 1, **caractérisée par le fait que** l'acide carboxylique insaturé est choisi parmi l'acide acrylique, l'acide méthacrylique, l'acide itaconique ou l'acide crotonique.

3. Composition selon la revendication 1 ou 2, **caractérisée par le fait que** l'acide carboxylique insaturé est l'acide acrylique.

4. Composition cosmétique selon l'une quelconque des revendications 1 à 3, **caractérisée par le fait que** le vinyl lactame est la vinylpyrrolidone.

5. Composition cosmétique selon l'une quelconque des revendications précédentes, **caractérisée par le fait que** les esters acrylique ou méthacrylique comportent un radical alkyle comportant de 8 à 18 atomes de carbone.

6. Composition cosmétique selon la revendication 5, **caractérisée par le fait que** les radicaux alkyle des esters acrylique ou méthacrylique sont choisis parmi les radicaux 2-éthylhexyle, octyle, lauryle et stéaryle.

7. Composition cosmétique selon l'une quelconque des revendications précédentes, **caractérisée par le fait que** ledit polymère fixant anionique comprend en tant que monomères de la vinylpyrrolidone, de l'acide acrylique et du méthacrylate de lauryle.

8. Composition cosmétique selon l'une quelconque des revendications précédentes, **caractérisée par le fait que** ledit polymère fixant est présent à une concentration allant de 0,05 à 30 % en poids, de préférence à une concentration allant de 0,1 à 20 % en poids, et encore plus préférentiellement à une concentration allant de 0,5 à 10 % en poids, par rapport au poids total de la composition.

9. Composition cosmétique selon la revendication 1, **caractérisée par le fait que** le poly(acide 2-acrylamido 2-méthyl propane sulfonique) réticulé comporte de 98 à 99,5 % en poids de motifs de formule (I) et de 0,2 à 2 % en poids de motifs réticulants.

10. Composition cosmétique selon l'une quelconque des revendications précédentes, **caractérisée par le fait que** le poly(acide 2-acrylamido 2-méthyl propane sulfonique) est réticulé par le triméthylol propane triacrylate.

11. Composition cosmétique selon l'une quelconque des revendications précédentes, **caractérisée par le fait que** le poly(acide 2-acrylamido 2-méthyl propane sulfonique) réticulé présente une viscosité mesurée au viscosimètre Brookfield, mobile 4, à une vitesse de rotation de 100 tours/minutes, dans une solution d'eau à 2 % et à 25°C, supérieure ou égale à 1 Pa.s (1000cps).

12. Composition cosmétique selon l'une quelconque des revendications précédentes, **caractérisée par le fait que** le poly(acide 2-acrylamido 2-méthyl propane sulfonique) réticulé présente une viscosité mesurée au viscosimètre Brookfield, mobile 4, à une vitesse de rotation de 100 tours/minutes, dans une solution d'eau à 2 % et à 25°C, allant de 5 Pa.s à 40 Pa.s (5000 à 40000cps) et plus particulièrement de 6,5 Pa.s à 35 Pa.s (6500 à 35000 cps).

13. Composition cosmétique selon l'une quelconque des revendications précédentes, **caractérisée par le fait que** le ou les polymères sulfonés sont présents à une concentration allant de 0,05 à 20 % en poids, de préférence de 0,1 à 10% en poids, et encore plus préférentiellement de 0,5 à 5 % en poids par rapport au poids total de la composition.

14. Composition selon l'une quelconque des revendications précédentes, **caractérisée par le fait qu'**elle présente une viscosité comprise entre 0,2 et 100 Pa.s (2 et 1000 poise) à 25°C (200 et 100000 cps), de préférence entre 0,5 et 50 Pa.s (5 et 500 poise) à 25°C (500 et 50000 cps), et encore plus préférentiellement entre 0,18 et 30 Pa.s (8 et 300 poise) à 25°C (800 et 30000 cps) à un taux de cisaillement de 1s⁻¹.

15. Composition cosmétique selon l'une quelconque des revendications précédentes, **caractérisée par le fait que** le rapport pondéral entre le ou les polymères fixants anioniques et le ou les polymères sulfonés est supérieur à 1.

16. Composition selon l'une quelconque des revendications précédentes, **caractérisée par le fait qu'**elle comprend en outre au moins un adjuvant cosmétique choisi parmi les polymères fixants autre que ceux de l'invention, les silicones sous forme soluble, dispersées, micro ou nano-dispersées, les agents épaississants non polymériques, des polymères épaississants ou agents gélifiants non sulfonés, les agents tensioactifs cationiques, anioniques, amphotères, non ioniques, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents anticorrosion, les corps gras tels que les huiles végétales, animales, minérales et synthétiques, les agents réducteurs ou antioxydants, les agents oxydants.

17. Composition cosmétique selon la revendication 16, **caractérisée par le fait que** le ou les adjuvants cosmétiques sont présents à une concentration allant de 0,001 à 50 % en poids par rapport au poids total de la composition.

18. Composition cosmétique selon l'une quelconque des revendications précédentes, **caractérisée par le fait que** le milieu cosmétiquement acceptable est un milieu aqueux, alcoolique ou hydroalcoolique.

19. Composition cosmétique selon la revendication 18, **caractérisée par le fait que** le milieu hydroalcoolique comprend les alcools inférieurs en C₁-C₄, les polyols, les monoéthers de polyols et leurs mélanges.

20. Composition cosmétique selon la revendication 19, **caractérisée par le fait que** l'alcool est de l'éthanol.

21. Composition cosmétique selon l'une quelconque des revendications précédentes, **caractérisée par le fait qu'**elle est conditionnée dans un vaporisateur, un flacon pompe ou un dispositif aérosol.

22. Composition cosmétique selon la revendication 21, **caractérisée par le fait qu'**elle est conditionnée dans un dispositif aérosol.

23. Composition cosmétique selon la revendication 22, **caractérisée par le fait qu'**elle comprend un agent propulseur choisi parmi l'air, l'azote, le gaz carbonique, le diméthyléther, les alcanes en C₃ à C₅, le 1,1-difluoroéthane et leurs mélanges.

24. Dispositif aérosol formé par un récipient comprenant une composition selon l'une quelconque des revendications précédentes ainsi qu'un moyen de distribution de la composition.

25. Procédé de traitement cosmétique **caractérisée par le fait qu'**il comprend l'application d'une composition selon l'une quelconque des revendications 1 à 23, en particulier sur les cheveux.

26. Procédé de traitement cosmétique selon la revendication 25, **caractérisée par le fait que** l'application de ladite composition n'est pas suivie par un rinçage.

27. Utilisation d'une composition cosmétique selon l'une quelconque des revendications 1 à 23 pour la mise en forme et/ou le maintien de la coiffure.

## Claims

1. Cosmetic composition for treating keratin fibres, especially human keratin fibres such as the hair, **characterized in that** it comprises, in a cosmetically acceptable medium,
- at least one anionic fixative polymer comprising at least as monomers a vinyl lactam, an ethylenically unsaturated carboxylic acid and an alkyl acrylate or methacrylate whose alkyl moiety contains at least 6 carbon atoms, and
- at least one partially or fully neutralized sulphonated polymer containing at least one unit derived from a sulpho-functional ethylenically unsaturated monomer, said sulphonated polymer being selected from a poly(2-acrylamido-2-methylpropanesulphonic acid) crosslinked and at least 90% neutralized comprising:
(a) from 90% to 99.9% by weight of units of general formula (I): 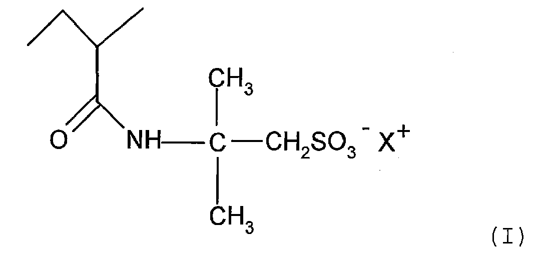 in which X⁺ is the ammonium ion;
(b) from 0.01% to 10% by weight of crosslinking units originating from at least one monomer having at least two olefinic double bonds; the proportions by weight being defined in relation to the total weight of the polymer.

2. Cosmetic composition according to Claim 1, **characterized in that** the unsaturated carboxylic acid is selected from acrylic acid, methacrylic acid, itaconic acid and crotonic acid.

3. Composition according to Claim 1 or 2, **characterized in that** the unsaturated carboxylic acid is acrylic acid.

4. Cosmetic composition according to any one of Claims 1 to 3, **characterized in that** the vinyl lactam is vinylpyrrolidone.

5. Cosmetic composition according to any one of the preceding claims, **characterized in that** the acrylic or methacrylic esters contain an alkyl radical containing 8 to 18 carbon atoms.

6. Cosmetic composition according to Claim 5, **characterized in that** the alkyl radicals of the acrylic or methacrylic esters are selected from 2-ethylhexyl, octyl, lauryl and stearyl radicals.

7. Cosmetic composition according to any one of the preceding claims, **characterized in that** the said anionic fixative polymer comprises as monomers vinylpyrrolidone, acrylic acid and lauryl methacrylate.

8. Cosmetic composition according to any one of the preceding claims, **characterized in that** the said fixative polymer is present at a concentration ranging from 0.05% to 30% by weight, preferably at a concentration ranging from 0.1% to 20% by weight, and more preferentailly at a concentration ranging from 0.5% to 10% by weight, relative to the total weight of the composition.

9. Cosmetic composition according to Claim 1, **characterized in that** the crosslinked poly(2-acrylamido-2-methylpropanesulphonic acid) contains from 98% to 99.5% by weight of units of formula (I) and from 0.2% to 2% by weight of crosslinking units.

10. Cosmetic composition according to any one of the preceding claims, **characterized in that** the poly(2-acrylamido-2-methylpropanesulphonic acid) is crosslinked by trimethylolpropane triacrylate.

11. Cosmetic composition according to any one of the preceding claims, **characterized in that** the crosslinked poly(2-acrylamido-2-methylpropanesulphonic acid) exhibits a viscosity as measured in a Brookfield viscometer using spindle 4 at a rotary speed of 100 revolutions/minute in 2% aqueous solution at 25°C of greater than or equal to 1 Pa.s (1000 cps).

12. Cosmetic composition according to any one of the preceding claims, **characterized in that** the crosslinked poly(2-acrylamido-2-methylpropanesulphonic acid) exhibits a viscosity as measured in a Brookfield viscometer using spindle 4 at a rotary speed of 100 revolutions/minute in 2% aqueous solution at 25°C ranging from 5 Pa.s to 40 Pa.s (5000 to 40 000 cps) and more particularly from 6.5 Pa.s to 35 Pa.s (6500 to 35 000 cps).

13. Cosmetic composition according to any one of the preceding claims, **characterized in that** the sulphonated polymer or polymers are present at a concentration ranging from 0.05% to 20% by weight, preferably from 0.1% to 10% by weight and more preferentially from 0.5% to 5% by weight relative to the total weight of the composition.

14. Composition according to any one of the preceding claims, **characterized in that** it exhibits a viscosity of between 0.2 and 100 Pa.s (2 and 1000 poise) at 25°C (200 and 100 000 cps), preferably between 0.5 and 50 Pa.s (5 and 500 poise) at 25°C (500 and 50 000 cps) and more preferentially between 0.8 and 30 Pa.s (8 and 300 poise) at 25°C (800 and 30 000 cps) and a shear rate of 1 s⁻¹.

15. Cosmetic composition according to any one of the preceding claims, **characterized in that** the weight ratio between the anionic fixative polymer or polymers and the sulphonated polymer or polymers is greater than 1.

16. Composition according to any one of the preceding claims, **characterized in that** it further comprises at least one cosmetic adjuvant selected from fixative polymers other than those of the invention, dispersed, microdispersed or nanodispersed silicones in soluble form, non-polymeric thickeners, thickening polymers or gelling agents which are not sulphonated, cationic, anionic, amphoteric and nonionic surfactants, ester-type conditioning agents, antifoams, moisturizers, emollients, plasticizers, water-soluble and fat-soluble silicone or non-silicone sunscreens, permanent or temporary dyes, fragrances, peptizing agents, preservatives, ceramides and pseudoceramides, vitamins and provitamins including panthenol, proteins, sequestrants, solubilizers, alkalifiers, anticorrosion agents, fatty substances such as vegetable, animal, mineral and synthetic oils, reducing agents or antioxidants, and oxidizing agents.

17. Cosmetic composition according to Claim 16, **characterized in that** the cosmetic adjuvant or adjuvants are present at a concentration ranging from 0.001% to 50% by weight relative to the total weight of the composition.

18. Cosmetic composition according to any one of the preceding claims, **characterized in that** the cosmetically acceptable medium is an aqueous, alcoholic or aqueous-alcoholic medium.

19. Cosmetic composition according to Claim 18, **characterized in that** the aqueous-alcoholic medium comprises C₁-C₄ lower alcohols, polyols, polyol monoethers and mixtures thereof.

20. Cosmetic composition according to Claim 19, **characterized in that** the alcohol is ethanol.

21. Cosmetic composition according to any one of the preceding claims, **characterized in that** it is packaged in a vaporizer, a pump flask or an aerosol device.

22. Cosmetic composition according to Claim 21, **characterized in that** it is packaged in an aerosol device.

23. Cosmetic composition according to Claim 22, **characterized in that** it comprises a propellant selected from air, nitrogen, carbon dioxide, dimethyl ether, C₃ to C₅ alkanes, 1,1-difluoroethane and mixtures thereof.

24. Aerosol device formed by a container comprising a composition according to any one of the preceding claims and also a means of distributing the composition.

25. Method of cosmetic treatment, **characterized in that** it comprises applying a composition according to any one of Claims 1 to 23 to the hair in particular.

26. Cosmetic treatment method according to Claim 25, **characterized in that** the application of said composition is not followed by rinsing.

27. Use of a cosmetic composition according to any one of Claims 1 to 23 for shaping and/or retaining the hairstyle.

## Patentansprüche

1. Kosmetische Zusammensetzung zur Behandlung von Keratinfasern, insbesondere menschlichen Keratinfasern wie den Haaren, **dadurch gekennzeichnet, dass** sie in einem kosmetisch unbedenklichen Medium
- mindestens ein anionisches fixierendes Polymer, das mindestens als Monomere ein Vinyllactam, eine ethylenisch ungesättigte Carbonsäure und ein Alkylacrylat oder -methacrylat mit mindestens 6 Kohlenstoffatomen im Alkylteil umfasst, und
- mindestens ein teilweise oder vollständig neutralisiertes sulfoniertes Polymer, das mindestens eine Einheit, die sich von einem ethylenisch ungesättigten Monomer mit einer Sulfogruppe ableitet, umfasst, wobei das sulfonierte Polymer aus einer vernetzten und zu mindestens 90% neutralisierten Poly(2-acrylamido-2-methylpropan-sulfonsäure), umfassend:
(a) 90 bis 99,9 Gew.-% Einheiten der folgenden allgemeinen Formel (I): 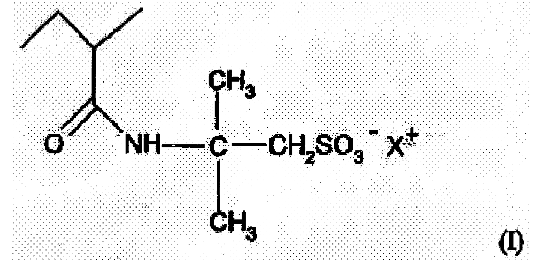 worin X⁺ für das Ammoniumion steht;
(b) 0,01 bis 10 Gew.-% Vernetzungseinheiten, die aus mindestens einem Monomer mit mindestens zwei olefinischen Doppelbindungen stammen; wobei die Gewichtsanteile in Bezug auf das Gesamtgewicht des Polymers definiert sind;
ausgewählt ist;
umfasst.

2. Kosmetische Zusammensetzung nach Anspruch 1, **dadurch gekennzeichnet, dass** die ungesättigte Carbonsäure aus Acrylsäure, Methacrylsäure, Itaconsäure und Crotonsäure ausgewählt ist.

3. Kosmetische Zusammensetzung nach Anspruch 1 oder 2, **dadurch gekennzeichnet, dass** es sich bei der ungesättigten Carbonsäure um Acrylsäure handelt.

4. Kosmetische Zusammensetzung nach einem der Ansprüche 1 bis 3, **dadurch gekennzeichnet, dass** es sich bei dem Vinyllactam um Vinylpyrrolidon handelt.

5. Kosmetische Zusammensetzung nach einem der vorhergehenden Ansprüche, **dadurch gekennzeichnet, dass** die Acryl- oder Methacrylsäureester einen Alkylrest mit 8 bis 18 Kohlenstoffatomen enthalten.

6. Kosmetische Zusammensetzung nach Anspruch 5, **dadurch gekennzeichnet, dass** die Alkylreste der Acryl- oder Methacrylsäureester aus 2-Ethylhexyl-, Octyl-, Lauryl- und Stearylresten ausgewählt sind.

7. Kosmetische Zusammensetzung nach einem der vorhergehenden Ansprüche, **dadurch gekennzeichnet, dass** das anionische fixierende Polymer als Monomere Vinylpyrrolidon, Acrylsäure und Laurylmethacrylat umfasst.

8. Kosmetische Zusammensetzung nach einem der vorhergehenden Ansprüche, **dadurch gekennzeichnet, dass** das fixierende Polymer in einer Konzentration im Bereich von 0,05 bis 30 Gew.-%, vorzugsweise bei einer Konzentration im Bereich von 0,1 bis 20 Gew.-% und noch weiter bevorzugt bei einer Konzentration im Bereich von 0,5 bis 10 Gew.-%, bezogen auf das Gesamtgewicht der Zusammensetzung, vorliegt.

9. Kosmetische Zusammensetzung nach Anspruch 1, **dadurch gekennzeichnet, dass** die vernetzte Poly(2-acrylamido-2-methylpropansulfonsäure) 98 bis 99,5 Gew.-% Einheiten der Formel (I) und 0,2 bis 2 Gew.-% Vernetzungseinheiten enthält.

10. Kosmetische Zusammensetzung nach einem der vorhergehenden Ansprüche, **dadurch gekennzeichnet, dass** die Poly(2-acrylamido-2-methylpropansulfonsäure) durch Trimethylolpropantriacrylat vernetzt ist.

11. Kosmetische Zusammensetzung nach einem der vorhergehenden Ansprüche, **dadurch gekennzeichnet, dass** die vernetzte Poly(2-acrylamido-2-methyl-propansulfonsäure) eine auf einem Brookfield-Viskosimeter, Spindel 4, bei einer Rotationsgeschwindigkeit von 100 Umdrehungen/Minute in 2%iger wässriger Lösung bei 25°C gemessene Viskosität größer gleich 1 Pa.s (1000 cP) aufweist.

12. Kosmetische Zusammensetzung nach einem der vorhergehenden Ansprüche, **dadurch gekennzeichnet, dass** die vernetzte Poly(2-acrylamido-2-methylpropansulfonsäure) eine auf einem Brookfield-Viskosimeter, Spindel 4, bei einer Rotationsgeschwindigkeit von 100 Umdrehungen/Minute in 2%iger wässriger Lösung bei 25°C gemessene Viskosität von 5 Pa.s bis 40 Pa.s (5000 bis 40.000 cP) und spezieller 6,5 Pa.s bis 35 Pa.s (6500 bis 35.000 cP) aufweist.

13. Kosmetische Zusammensetzung nach einem der vorhergehenden Ansprüche, **dadurch gekennzeichnet, dass** das oder die sulfonierten Polymere in einer Konzentration im Bereich von 0,05 bis 20 Gew.-%, vorzugsweise von 0,1 bis 10 Gew.-% und noch weiter bevorzugt von 0,5 bis 5 Gew.-%, bezogen auf das Gesamtgewicht der Zusammensetzung, vorliegen.

14. Kosmetische Zusammensetzung nach einem der vorhergehenden Ansprüche, **dadurch gekennzeichnet, dass** sie eine Viskosität zwischen 0,2 und 100 Pa.s (2 und 1000 Poise) bei 25°C (200 und 100.000 cP), vorzugsweise zwischen 0,5 und 50 Pa.s (5 und 500 Poise) bei 25°C (500 und 50.000 cP) und noch weiter bevorzugt zwischen 0,8 und 30 Pa.s (8 und 300 Poise) bei 25°C (800 und 30.000 cP) bei einer Scherrate von 1 s⁻¹ aufweist.

15. Kosmetische Zusammensetzung nach einem der vorhergehenden Ansprüche, **dadurch gekennzeichnet, dass** das Gewichtsverhältnis zwischen dem oder den anionischen fixierenden Polymeren und dem oder den sulfonierten Polymeren größer als 1 ist.

16. Kosmetische Zusammensetzung nach einem der vorhergehenden Ansprüche, **dadurch gekennzeichnet, dass** sie außerdem mindestens einen kosmetischen Hilfsstoff, der aus anderen fixierenden Polymeren als denjenigen der Erfindung, Silikonen in gelöster, dispergierter, mikrodispergierter oder nanodispergierter Form, nichtpolymeren Verdickern, verdickend wirkenden Polymeren oder nicht sulfonierten Gelierungsmitteln, kationischen, anionischen, amphoteren und nichtionischen Tensiden, Konditionierungsmitteln vom Ester-Typ, Antischaummitteln, feuchtigkeitsspendenden Mitteln, Emollientien, Weichmachern, wasserlöslichen und fettlöslichen Silikon- und Nichtsilikon-Lichtschutzmitteln, permanenten und temporären Farbstoffen, Duftstoffen, Peptisierungsmitteln, Konservierungsstoffen, Ceramiden, Pseudoceramiden, Vitaminen und Provitaminen, darunter Panthenol, Proteinen, Sequestriermitteln, Solubilisierungsmitteln, Alkalinisierungsmitteln, Korrosionsschutzmitteln, Fettsubstanzen wie pflanzlichen, tierischen, mineralischen und synthetischen Ölen, Reduktionsmitteln oder Antioxidantien und Oxidationsmitteln ausgewählt ist, umfasst.

17. Kosmetische Zusammensetzung nach Anspruch 16, **dadurch gekennzeichnet, dass** der oder die kosmetischen Hilfsstoffe in einer Konzentration im Bereich von 0,001 bis 50 Gew.-%, bezogen auf das Gesamtgewicht der Zusammensetzung, vorliegen.

18. Kosmetische Zusammensetzung nach einem der vorhergehenden Ansprüche, **dadurch gekennzeichnet, dass** es sich bei dem kosmetisch unbedenklichen Medium um ein wässriges, alkoholisches oder wässrig-alkoholisches Medium handelt.

19. Kosmetische Zusammensetzung nach Anspruch 18, **dadurch gekennzeichnet, dass** das wässrig-alkoholische Medium C₁-C₄-Niederalkohole, Polyole, Polyolmonoether und Mischungen davon umfasst.

20. Kosmetische Zusammensetzung nach Anspruch 19, **dadurch gekennzeichnet, dass** es sich bei dem Alkohol um Ethanol handelt.

21. Kosmetische Zusammensetzung nach einem der vorhergehenden Ansprüche, **dadurch gekennzeichnet, dass** sie in einem Zerstäuber, einer Pumpflasche oder einer Aerosolvorrichtung verpackt ist.

22. Kosmetische Zusammensetzung nach Anspruch 21, **dadurch gekennzeichnet, dass** sie in einer Aerosolvorrichtung verpackt ist.

23. Kosmetische Zusammensetzung nach Anspruch 22, **dadurch gekennzeichnet, dass** sie ein Treibmittel, das aus Luft, Stickstoff, Kohlendioxid, Dimethylether, C₃- bis C₅-Alkanen, 1,1-Difluorethan und Mischungen davon ausgewählt ist, umfasst.

24. Aerosolvorrichtung, gebildet aus einem Behältnis, das eine Zusammensetzung nach einem der vorhergehenden Ansprüche umfasst, sowie einem Mittel zum Verteilen der Zusammensetzung.

25. Verfahren zur kosmetischen Behandlung, **dadurch gekennzeichnet, dass** man eine Zusammensetzung nach einem der Ansprüche 1 bis 23 aufbringt, insbesondere auf die Haare.

26. Verfahren zur kosmetischen Behandlung nach Anspruch 25, **dadurch gekennzeichnet, dass** nach dem Aufbringen der Zusammensetzung nicht gespült wird.

27. Vewendung einer Zusammensetzung nach einem der Ansprüche 1 bis 23 zum Gestalten und/oder Festigen der Frisur.
